Europäisches Patentamt

European Patent Office

Office européen des brevets

(19)

(11) Publication number: **0 438 183 B1**

(12) **EUROPEAN PATENT SPECIFICATION**

(45) Date of publication of patent specification: **08.11.95**

(51) Int. Cl.[6]: **A61K 31/71, A61K 9/08**

(21) Application number: **91102986.6**

(22) Date of filing: **03.12.87**

(60) Publication number of the earlier application in accordance with Art.76 EPC: **0 273 603**

Divisional application 95101919.9 filed on 03/12/87.

(54) **Injectable ready-to-use solutions containing an antitumor anthracycline glycoside.**

(30) Priority: **05.12.86 GB 8629193**
**22.06.87 US 64653**

(43) Date of publication of application:
**24.07.91 Bulletin 91/30**

(45) Publication of the grant of the patent:
**08.11.95 Bulletin 95/45**

(84) Designated Contracting States:
**AT BE CH DE ES FR GB GR IT LI LU NL SE**

(56) References cited:
**FR-A- 2 405 957**
**GB-A- 2 178 311**

**JOURNAL OF PARENTERAL SCIENCE AND TECHNOLOGY, vol. 39, no. 6, November-December1985, pages 220-222; J.H. BEIJNEN et al.: "Stability of anthracycline antitumor agents in infusion fluids".**

**THE MERCK INDEX, 10th edition, 1983, page 499, edited by M. WINDHOLZ et al.,Merck & Co., Rahway, US, abstract no. 3435: "Doxorubicin"**

(73) Proprietor: **PHARMACIA S.p.A.**
**Via Robert Koch, 1.2**
**I-20152 Milano (IT)**

(72) Inventor: **Gatti, Gaetano**
**Viale Fulvio Testi 42**
**Sesto San Giovanni,**
**Milan (IT)**
Inventor: **Oldani, Diego**
**Via San Giovanni 48**
**Robecco sul Naviglio,**
**Milan (IT)**
Inventor: **Bottoni, Giuseppe**
**Via Don Luigi Guanella 5**
**I-24100 Bergamo (IT)**
Inventor: **Confalonieri, Carlo**
**Via Ticino 5**
**Cusano Milanino (Milan) (IT)**
Inventor: **Gambini, Luciano**
**Via Piave 16**
**Cornaredo (Milan) (IT)**
Inventor: **De Ponti, Roberto**
**Via degli Astri 22**
**Milan (IT)**

EP 0 438 183 B1

(74) Representative: **Woods, Geoffrey Corlett et al**
**J.A. KEMP & CO.**
**14 South Square**
**Gray's Inn**
**London WC1R 5LX (GB)**

## Description

The present invention relates to a storage stable, injectable ready-to-use solution of an antitumor anthracycline glycoside and to a process for preparing such a solution.

The anthracycline glycoside compounds are a well known class of compounds in the antineoplastic group of agents, of which doxorubicin is a typical, and the most widely used, representative: Doxorubicin. Anticancer Antibiotics, Federico Arcamone, 1981, Publ: Academic Press, New York, N.Y.; Adriamycin Review, EROTC international Symposium, Brussels, May, 1974, edited by M. Staquet, Publ. Eur. Press Medikon, Ghent, Belg.; and Results of Adriamycin Therapy, Adriamycin Symposium at Frankfurt/Main 1974 edited by M.Ghione, J.Fetzer and H.Maier, publ.: Springer, New York, N.Y.

At present, anthracycline glycoside antitumor drugs, in particular, e.g. doxorubicin, are solely available in the form of lyophilized preparations, which need to be reconstituted before administration. Both the manufacture and the reconstitution of such preparations expose the involved personnel (workers, pharmacists, medical personnel, nurses) to risks of contamination which are particularly serious due to the toxicity of the antitumor substances.

The Martindale Extra Pharmacopoeia 28th edition, page 175 left column, reports on adverse effects of antineoplastic drugs and recommends that "They must be handled with great care and contact with skin and eyes avoided; they should not be inhaled. Care must be taken to avoid extravasation since pain and tissue damage may ensue.". Similarly, Scand. J. Work Environ Health vol.10 (2), pages 71-74 (1984), as well as articles in Chemistry Industry, Issue July 4, 1983, page 488, and Drug-Topics-Medical-Economics-Co,Issue February 7, 1983, page 99 report about severe adverse effects observed in medical personnel exposed to use of cytostatic agents, including doxorubicin.

To administer a lyophilized preparation, double handling of the drug is required. The lyophilized cake first has to be reconstituted and then administered. Moreover, in some cases, the complete dissolution of the powder may require prolonged shaking because of solubilization problems. Reconstitution of a lyophilized cake or powder can result in formation of aerosol droplets which can be inhaled or can come into contact with skin or mucous membranes of those handling the solution.

As the risks connected with the manufacture and the reconstitution of a lyophilized preparate would be highly reduced if a ready-to-use solution of the drug were available, we have developed a stable, therapeutically acceptable intravenously injectable solution of an anthracycline glycoside drug, e.g. doxorubicin, whose preparation and administration does not require either lyophilization or reconstitution.

GB-A-2178311 describes and claims sterile, pyrogen-free, anthracycline glycoside solutions which consist essentially of a physiologically acceptable salt of an anthracycline glycoside such as doxorubicin dissolved in a physiologically acceptable solvent therefor, which have not been reconstituted from a lyophilizate and which have a pH of from 2.5 to 6.5. Especially preferred pH's are about 3 and about 5. The Examples illustrate solutions with pH's ranging from 2.62 to 3.14, with pH 4.6 and with pH 5.2. There is no mention of stabilising agents. Dextrose, lactose and mannitol are mentioned as tonicity adjustment agents but no indication is given of the proportions in which they may be used.

The present invention provides a storage stable, sterile, pyrogen-free, ready-to-use, injectable 4'-epi-doxorubicin solution which is sealed in a container, which consists essentially of 4'-epi-doxorubicin hydrochloride dissolved in a physiologically acceptable aqueous solvent therefor at a concentration of 4'-epi-doxorubicin of from 0.1 mg/ml to 50 mg/ml, which has not been reconstituted from a lyophilizate and the pH of which has been adjusted to from 2.5 to 4.0 solely by means of a physiologically acceptable acid.

It is thus possible to provide solutions of 4'-epi-doxorubicin (i.e. epirubicin) which are storage stable and have a commercially meaningful shelf-like.

The solution of the invention is provided in a sealed container, especially one made of glass. The solution can be provided in this way either in a unit dosage form or in a multiple dosage form.

Any aqueous solvent which is physiologically acceptable and which is able to dissolve the epirubicin salt may be used. The solution of the invention may also contain one or more formulation adjuvants such as a co-solubilizing agent (which may be the same as a solvent), a tonicity adjustment agent, a preservative and a pharmaceutically acceptable chelating agent.

Suitable solvents and co-solubilizing agents may be, for instance, water e.g. Water for Injections; a 0.9 % sodium chloride solution, i.e. physiological saline; an aqueous 5 % dextrose solution; and mixtures of water and one or more of:
- an aliphatic amide, e.g. N,N-dimethylacetamide, N-hydroxy-2-ethyl-lactamide and the like;
- an alcohol, e.g. ethanol, benzyl alcohol and the like;
- a glycol or polyalcohol, e.g. propyleneglycol, glycerin and the like;
- an ester of a polyalcohol, e.g. diacetine, triacetine and the like;

EP 0 438 183 B1

- a polyglycol or a polyether, e.g. polyethyleneglycol 400, propyleneglycol methylethers and the like;
- a dioxolane, e.g. isopropylidenglycerin and the like;
- dimethylisosorbide; and
- a pyrrolidone derivative, e.g. 2-pyrrolidone, N-methyl-2-pyrrolidone, polyvinylpyrrolidone and the like.

Examples of preferred solvents are water, physiological saline, an aqueous 5 % dextrose solution and mixtures of water and one or more of ethanol, polyethyleneglycol and dimethylacetamide. Water, physiological saline and a 5 % dextrose solution are particularly preferred.

Suitable tonicity adjustment agents may be, for instance, physiologically acceptable inorganic chlorides, e.g. sodium chloride (preferably 0.9 % by weight sodium chloride), dextrose, lactose, mannitol, sorbitol and the like.

Preservatives suitable for physiological administration may be, for instance, esters of para-hydroxybenzoic acid (e.g., methyl, ethyl, propyl and butyl esters, or mixtures of them), chlorocresol and the like.

A suitable pharmaceutically acceptable chelating agent may be ethylenediaminotetraacetic acid (EDTA). The chelating agent is included in a minor amount, typically from 0.001 to 0.05 % by weight.

The above mentioned solvents tonicity adjustment agents, preservatives and chelating agents can be used alone or as a mixture of two or more of them.

To adjust the pH within the range of from 2.5 to 4.0 a physiologically acceptable acid is added as desired. The acid may be any physiologically acceptable acid, e.g. an inorganic mineral acid such as hydrochloric, sulfuric, phosphoric and the like, or an organic acid such as acetic, succinic, tartaric, ascorbic, citric, glutamic, methanesulphonic, ethanesulfonic and the like.

The preferred range of pH for the ready-to-use solution of the invention is from 2.5, e.g. from about 2.6, to about 3.7, e.g. to about 3.5. A more preferred pH range is from about 3 to about 3.5. A pH of about 3 is particularly preferred. Other preferred pH ranges are from greater than 3.14, e.g. from about 3.2, to 4 and preferably to about 3.7, more preferably to about 3.5. A useful solution with a pH of from 2.62 to 3.14 further comprises: a pharmaceutically acceptable chelating agent.

In the solutions of the invention the concentration of epirubicin varies from 0.1 mg/ml to 50 mg/ml, preferably from 1 mg/ml to 20 mg/ml. Other preferred ranges of concentration are from about 2 mg/ml to about 50 mg/ml, preferably from 2 mg/ml to 20 mg/ml, particularly appropriate values being 2 mg/ml and 5 mg/ml.

Suitable packaging for the epirubicin solutions may be all approved containers intended for parenteral use, such as plastic and glass containers, ready-to-use syringes and the like. Preferably the container is a sealed glass container, e.g. a vial or an ampoule. A hermetically sealed glass vial is particularly preferred.

The invention also provides a process for producing a storage stable, sterile, pyrogen-free, ready-to-use, injectable solution according to the invention, which process comprises

(i) dissolving the epirubicin hydrochloride, which is not in the form of a lyophilizate, in the physiologically acceptable aqueous solvent therefor at a concentration of epirubicin of from 0.1 mg/ml to 50 mg/ml;

(ii) optionally, adding one or more formulation adjuvants selected from co-solubilizing agents, tonicity adjustment agents, preservatives and pharmaceutically acceptable chelating agents;

(iii) adding solely a physiologically acceptable acid to adjust the pH to from 2.5 to 4.0 as desired;

(iv) sealing the solution in a container; and the process being effected in such a manner that the resultant solution is sterile and pyrogen-free.

Any suitable procedure may be adopted to ensure that the resultant solution is sterile and pyrogen-free. Preferably, the solution is passed through a sterilising filter after step (iii) but before step (iv) although of course one or more of the materials used may be sterile and pyrogen-free anyway. Where all materials employed are sterile and pyrogen-free, there may then be no need for passing the resultant solution through a sterilising filter.

With the solutions of the invention it is possible to obtain compositions having a very high concentration of the active substance even at 50 mg/ml. This constitutes a great advantage over the presently available lyophilized preparates wherein high concentrations can only be obtained with difficulty because of solubilization problems encountered in reconstitution, mainly with saline. The presence of the excipient, e.g. lactose, in the lyophilized cake, and its generally high proportion in respect of the active substance, even up to 5 parts of excipient per part of active substance, has a negative effect on solubilization so that difficulties may arise in obtaining dissolution of the lyophilized cake, especially for concentrations of epirubicin higher than 2 mg/ml.

The solutions of the invention are characterized by a good stability. Solutions in various solvents and with different pH's and concentrations have been found to be stable for long periods at temperatures accepted for the storage of pharmaceutical preparations. This is illustrated in the Examples which follow.

4

Owing to the well known anti-tumor activity of the active drug substance, the pharmaceutical compositions of the invention are useful for treating tumors in both human and animal hosts. Examples of tumors that can be treated are, for instance, sarcomas, including osteogenic and soft tissue sarcomas, carcinomas, e.g., breast-, lung-, bladder-, thyroid-, prostate- and ovarian carcinoma, lymphomas, including Hodgkin and non-Hodgkin lymphomas, neuroblastoma, melanoma, myeloma, Wilms tumor, and leukemias, including acute lymphoblastic leukemia and acute myeloblastic leukemia.

Examples of specific tumours that can be treated are Moloney Sarcoma Virus, Sarcoma 180 Ascites, solid Sarcoma 180, gross transplantable leukemia, L 1210 leukemia and lymphocytic P 388 leukemia.

Inhibition of the growth of a tumour, in particular one of those indicated above, can be achieved by administering to a host suffering from a said tumour an injectable solution according to the invention containing the active drug substance in an amount sufficient to inhibit the growth of said tumour.

The injectable solutions of the invention are administered by rapid e.g. intravenous injection or infusion according to a variety of possible dose schedules.

Suitable dosages for 4'-epi-doxorubicin may be, for instance, of 75 to 90 mg/m$^2$ given in a single infusion to be repeated at 21 days.

The following Example illustrates the invention. A Reference Example is also provided.

Reference Example: Methodologies for long term stability of doxorubicin formulations having a pH of from 2.5 to 3.5

The formulations were prepared as follows.

Doxorubicin.HCl was dissolved in about 90 percent of the stated amount of water for injections or physiological saline, deaerated by nitrogen bubbling. Hydrochloric acid was added dropwise to adjust the pH of the solution. Deaerated water for injections or physiological saline was then added in order to obtain a doxorubicin.HCl concentration of 2 mg/ml. The solutions were filtered through a 0.22 $\mu$m microporous membrane under nitrogen pressure. Volumes of 5 ml, 10 ml and 25 ml of the solutions were distributed into type I, colourless glass vials having 8 or 10 ml, 14 ml and 39 ml top capacity, respectively. The vials were then closed with chlorobutyl teflon-faced rubber stoppers and sealed with aluminium caps. The formulations thus prepared were tested as regards appearance, clarity of solutions, pH, sterility (8°C, yearly), doxorubicin.HCl assay.

Test methods

For appearance and clarity: visual inspection
For pH: USP XXI
For sterility: USP XXI (membrane filtration)
For doxorubicin.HCl assay: HPLC ion-pair method and USP HPLC method (USP XXI)
Brief description of the HPLC ion-pair method for doxorubicin.HCl assay:

| | |
|---|---|
| Column filling | : reverse phase, Zorbax TMS |
| Mobile phase | : water: acetonitrile: methanol (54:29:17 v/v/v) containing 2 ml/l 85% phosphoric acid and 1 mg/ml sodium laurylsulfate (pairing agent) adjusted to pH 3.5 with 2N NaOH |
| Mobile phase flow rate | : 1.5 ml/min |
| Column temperature | : ambient (22°C ± 2°C) |
| Analytical wavelength | : 254 nm |
| System suitability parameters | : symmetry factor between 0.7 an 1.2; number of theoretical plates ≧ 2500; measurement reproducibility: variation coefficient <1, n = 6; resolution factor ≧ 12 |

The HPLC ion-pair method for doxorubicin.HCl assay is validated for accuracy, precision, linearity, sensitivity, specificity and stability-indicating nature.

Example: Stability of 4'-epi-doxorubicin (i.e.epirubicin) solutions

Solutions of epirubicin were prepared in the same fashion as the corresponding doxorubicin solutions hereinbefore. They were then tested for stability in the same way. The results are presented in Tables 1 to 24.

Table 1 - Stability studies. Batches tested in 0.9% Sodium Chloride Injection

| Batch No.<br>Batch characteristics | TF/23274 | TF/23275 | 6001LA |
|---|---|---|---|
| Epirubicin.HCl per vial (mg) | 10 | 10 | 10 |
| pH | 2.9 | 3.5 | 3.0 |
| Formulation No. | FI7701/IL2 | FI7701/IL2 | FI7701/IL2 |
| Batch size No of vials | 584 | 586 | 595 |
| Scale of manufacture * | Lab | Lab | S-i |
| Epirubicin.HCl batch No. | 6016G669 | 6016G669 | 6016G669 |

| Batch No.<br>Batch characteristics | 6001LB | 6001LC |
|---|---|---|
| Epirubicin.HCl per vial (mg) | 20 | 50 |
| pH | 3.1 | 2.7 |
| Formulation No. | FI7701/IL3 | FI7701/IL4 |
| Batch size No of vials | 500 | 760 |
| Scale of manufacture * | S-i | S-i |
| Epirubicin.HCl batch No. | 6017G703 | 6017G703 |

\* Lab = Laboratory
  S-i = Semi-industrial

Table 2 - Stability studies. Batches tested in 5% dextrose solution

| Batch No. Batch characteristics | TF/23270 | TF/23273 | 6001MA |
|---|---|---|---|
| Epirubicin.HCl per vial (mg) | 10 | 10 | 10 |
| pH | 2.7 | 3.5 | 2.9 |
| Formulation No. | FI7701/IL5 | FI7701/IL5 | FI7701/IL5 |
| Batch size No of vials | 564 | 573 | 553 |
| Scale of manufacture * | Lab | Lab | S-i |
| Epirubicin.HCl batch No. | 6016G669 | 6016G669 | 6016G669 |

| Batch No. Batch characteristics | 6001MB | 6001MC |
|---|---|---|
| Epirubicin.HCl per vial (mg) | 20 | 50 |
| pH | 3.1 | 2.6 |
| Formulation No. | FI7701/IL6 | FI7701/IL7 |
| Batch size No of vials | 500 | 751 |
| Scale of manufacture * | S-i | S-i |
| Epirubicin.HCl batch No. | 6017G703 | 6017G703 |

* Lab = Laboratory
  S-i = Semi-industrial

Table 3 - Stability studies. Batch tested in Water for Injection

| Batch No. Batch characteristics | TF/23176 |
|---|---|
| Epirubicin.HCl per vial (mg) | 10 |
| pH | 3.0 |
| Formulation No. | F17701/IL1 |
| Batch size No of vials | 430 |
| Scale of manufacture * | Laboratory |
| Epirubicin.HCl batch No. | 5009D646 |

Table 4 - Stability studies. Packaging used for epirubicin.HCl ready-to-use solution in 0.9% Sodium Chloride Injection

| Batch No. | TF/23274 | TF/23275 | 6001LA | 6001LB | 6001LC |
|---|---|---|---|---|---|
| Packaging | | | | | |
| vial glass type | I | I | I | I | I |
| vial top capacity | 10 ml | 10 ml | 10 ml | 14 ml | 39 ml |
| stopper | chlorobutyl rubber, teflon-faced | chlorobutyl rubber, teflon-faced | chlorobutyl rubber, teflon-faced | chlorobutyl rubber, teflon-faced | chlorobutyl rubber, teflon-faced |
| seal | aluminium | aluminium | aluminium | aluminium | aluminium |

Table 5 - Stability studies. Packaging used for epirubicin.HCl ready-to-use solution in 5% Dextrose

| Batch No.<br><br>Packaging | TF/23270 | TF/23273 | 6001MA | 6001MB | 6001MC |
|---|---|---|---|---|---|
| vial<br>glass type | I | I | I | I | I |
| vial<br>top capacity | 10 ml | 10 ml | 10 ml | 14 ml | 39 ml |
| stopper | chlorobutyl rubber,<br>teflon-faced | chlorobutyl rubber,<br>teflon-faced | chlorobutyl rubber,<br>teflon-faced | chlorobutyl rubber,<br>teflon-faced | chlorobutyl rubber,<br>teflon-faced |
| seal | aluminium | aluminium | aluminium | aluminium | aluminium |

**Table 6 — Stability studies – Packaging used for epirubicin.HCl ready-to-use in Water for Injection**

| Packaging | Batch No. | TF/23176 |
|---|---|---|
| vial glass type | | I |
| vial top capacity | | 10 ml |
| stopper | | chlorobutyl rubber, teflon-faced |
| seal | | aluminium |

EP 0 438 183 B1

Table 7 - Stability data of epirubicin.HCl ready-to-use

solution in 0.9% Sodium Chloride Injection, 4°C

<u>V i a l s   s t o r e d   u p r i g h t</u>

| Batch Dosage | | INITIAL CONTROL | 6 months |
|---|---|---|---|
| TF/23274 10 mg | • | 2.218 | |
| | •• | 100.0 | n.d. |
| | ••• | 2.7 | |
| | •••• | 2.9 | |
| TF/23275 10 mg | • | 2.223 | |
| | •• | 100.0 | n.d. |
| | ••• | 2.8 | |
| | •••• | 3.5 | |
| 6001LA 10 mg | • | 2.155 | 2.266 |
| | •• | 100.0 | 105.2 |
| | ••• | 1.9 | 3.3 |
| | •••• | 3.0 | 3.0 |
| 6001LB 20 mg | • | 1.961 | 2.013 |
| | •• | 100.0 | 102.6 |
| | ••• | 1.2 | 3.3 |
| | •••• | 3.1 | 3.1 |
| 6001LC 50 mg | • | 2.072 | 2.086 |
| | •• | 100.0 | 100.7 |
| | ••• | 1.4 | 3.0 |
| | •••• | 2.7 | 2.7 |

• epirubicin.HCl assay (mg/ml)
•• epirubicin.HCl % initial
••• related substances %
•••• pH

n.d. = not determined

Table 8 – Stability data of epirubicin.HCl ready-to-use solution in 0.9% Sodium Chloride Injection, 8°C

V i a l s   s t o r e d   u p r i g h t

| Batch Dosage | | INITIAL CONTROL | 1 months | 3 months | 6 months |
|---|---|---|---|---|---|
| TF/23274 10 mg | • •• ••• •••• | 2.218 100.0 2.7 2.9 | n.d. | n.d. | n.d. |
| TF/23275 10 mg | • •• ••• •••• | 2.223 100.0 2.8 3.5 | n.d. | n.d. | n.d. |
| 6001LA 10 mg | • •• ••• •••• | 2.155 100.0 1.9 3.0 | 2.165 100.5 3.8 3.0 | 2.143 99.4 2.9 3.0 | 2.182 101.3 3.7 3.0 |
| 6001LB 20 mg | • •• ••• •••• | 1.961 100.0 1.2 3.1 | 1.983 101.1 2.6 3.1 | 1.949 99.4 3.0 3.0 | n.d. |
| 6001LC 50 mg | • •• ••• •••• | 2.072 100.0 1.4 2.7 | 2.095 101.1 2.4 2.7 | 2.116 102.1 3.2 2.7 | 2.012 97.1 3.7 2.7 |

•    epirubicin.HCl assay (mg/ml)
••   epirubicin.HCl % initial
•••   related substances %
•••• pH

n.d. = not determined

Table 9 - Stability data of epirubicin.HCl ready-to-use
solution in 0.9% Sodium Chloride Injection, 4°C

V i a l s   s t o r e d   i n v e r t e d

| Batch Dosage | | INITIAL CONTROL | 6 months |
|---|---|---|---|
| TF/23274 10 mg | • •• ••• •••• | 2.218 100.0 2.7 2.9 | 2.247 101.3 4.1 2.9 |
| TF/23275 10 mg | • •• ••• •••• | 2.223 100.0 2.8 3.5 | 2.235 100.5 3.6 3.6 |
| 6001LA 10 mg | • •• ••• •••• | 2.155 100.0 1.9 3.0 | 2.219 103.0 3.5 3.0 |
| 6001LB 20 mg | • •• ••• •••• | 1.961 100.0 1.2 3.1 | 1.955 99.7 2.8 3.1 |
| 6001LC 50 mg | • •• ••• •••• | 2.072 100.0 1.4 2.7 | 2.154 104.0 3.0 2.7 |

•     epirubicin.HCl assay (mg/ml)
••    epirubicin.HCl % initial
•••   related substances %
••••  pH

Table 10 — Stability data of epirubicin.HCl ready-to-use solution in 0.9% Sodium Chloride Injection, 8°C

V i a l s    s t o r e d    i n v e r t e d

| Batch Dosage | | INITIAL CONTROL | 1 months | 3 months | 6 months |
|---|---|---|---|---|---|
| TF/23274 10 mg | • | 2.218 | 2.223 | 2.139 | 2.227 |
| | •• | 100.0 | 100.2 | 96.4 | 100.4 |
| | ••• | 2.7 | 2.5 | 3.9 | 3.7 |
| | •••• | 2.9 | 2.8 | 2.9 | 2.9 |
| TF/23275 10 mg | • | 2.223 | 2.226 | 2.121 | 2.228 |
| | •• | 100.0 | 100.1 | 95.4 | 100.2 |
| | ••• | 2.8 | 2.9 | 5.0 | 5.9 |
| | •••• | 3.5 | 3.5 | 3.5 | 3.5 |
| 6001LA 10 mg | • | 2.155 | 2.220 | 2.156 | 2.215 |
| | •• | 100.0 | 103.0 | 100.0 | 102.8 |
| | ••• | 1.9 | 3.6 | 3.3 | 3.6 |
| | •••• | 3.0 | 3.0 | 3.0 | 3.0 |
| 6001LB 20 mg | • | 1.961 | 2.019 | 1.951 | 1.980 |
| | •• | 100.0 | 102.9 | 99.5 | 101.0 |
| | ••• | 1.2 | 2.8 | 3.5 | 3.6 |
| | •••• | 3.1 | 3.1 | 3.0 | 3.1 |
| 6001LC 50 mg | • | 2.072 | 2.066 | 2.132 | 2.060 |
| | •• | 100.0 | 99.7 | 102.9 | 99.4 |
| | ••• | 1.4 | 2.8 | 3.3 | 3.2 |
| | •••• | 2.7 | 2.7 | 2.6 | 2.7 |

•    epirubicin.HCl assay (mg/ml)
••   epirubicin.HCl % initial
•••  related substances %
•••• pH

Table 11 - Stability date of epirubicin.HCl ready-to-use
solution in 0.9% Sodium Chloride Injection, 15°C

V i a l s   s t o r e d   i n v e r t e d

| Batch Dosage | | INITIAL CONTROL | 1 months | 3 months | 6 months |
|---|---|---|---|---|---|
| TF/23274 10 mg | • | 2.218 | 2.235 | 2.173 | 2.081 |
| | •• | 100.0 | 100.8 | 98.0 | 93.8 |
| | ••• | 2.7 | 4.1 | 3.9 | 5.4 |
| | •••• | 2.9 | 2.9 | 2.9 | 2.9 |
| TF/23275 10 mg | • | 2.223 | 2.221 | 2.108 | |
| | •• | 100.0 | 99.9 | 94.8 | n.d. |
| | ••• | 2.8 | 3.2 | 5.0 | |
| | •••• | 3.5 | 3.5 | 3.4 | |
| 6001LA 10 mg | • | 2.155 | 2.210 | 2.121 | 2.088 |
| | •• | 100.0 | 102.5 | 96.4 | 96.8 |
| | ••• | 1.9 | 3.4 | 3.5 | n.d. |
| | •••• | 3.0 | 3.0 | 3.0 | 3.0 |
| 6001LB 20 mg | • | 1.961 | 2.009 | 1.915 | |
| | •• | 100.0 | 102.4 | 97.6 | n.d. |
| | ••• | 1.2 | 3.6 | 3.7 | |
| | •••• | 3.1 | 3.1 | 3.0 | |
| 6001LC 50 mg | • | 2.072 | 2.052 | 2.081 | 2.036 |
| | •• | 100.0 | 99.0 | 100.4 | 98.3 |
| | ••• | 1.4 | 2.6 | 4.0 | 3.1 |
| | •••• | 2.7 | 2.7 | 2.6 | 2.7 |

- •    epirubicin.HCl assay (mg/ml)
- ••   epirubicin.HCl % initial
- •••  related substances %
- •••• pH

n.d. = not determined

Table 12 - Stability data of epirubicin.HCl ready-to-use solution in 0.9% Sodium Chloride Injection, 27°C

<u>Vials stored inverted</u>

| Batch Dosage | | INITIAL CONTROL | 1 month | 3 months |
|---|---|---|---|---|
| TF/23274 10 mg | • | 2.218 | 2.111 | 1.941 |
| | •• | 100.0 | 95.2 | 87.5 |
| | ••• | 2.7 | 5.4 | 7.1 |
| | •••• | 2.9 | 2.9 | 2.9 |
| TF/23275 10 mg | • | 2.223 | 2.041 | 1.729 |
| | •• | 100.0 | 91.8 | 77.8 |
| | ••• | 2.8 | 6.5 | 15.3 |
| | •••• | 3.5 | 3.5 | 3.2 |
| 6001LA 10 mg | • | 2.155 | 2.144 | 1.938 |
| | •• | 100.0 | 99.5 | 89.9 |
| | ••• | 1.9 | 5.2 | 9.9 |
| | •••• | 3.0 | 3.0 | 3.0 |
| 6001LB 20 mg | • | 1.961 | 1.867 | 1.738 |
| | •• | 100.0 | 95.7 | 88.8 |
| | ••• | 1.2 | 4.1 | 8.5 |
| | •••• | 3.1 | 3.1 | 3.0 |
| 6001LC 50 mg | • | 2.072 | 2.009 | 1.866 |
| | •• | 100.0 | 96.9 | 90.1 |
| | ••• | 1.4 | 4.1 | 9.1 |
| | •••• | 2.7 | 2.7 | 2.6 |

• epirubicin.HCl assay (mg/ml)
•• epirubicin.HCl % initial
••• related substances %
•••• pH

Table 13 —Stability data of epirubicin.HCl ready-to-use solution in 0.9% Sodium Chloride Injection, 250 foot candles

<u>V i a l s   s t o r e d   i n v e r t e d</u>

| Batch Dosage | | INITIAL CONTROL | 1 month | 3 months |
|---|---|---|---|---|
| TF/23274 10 mg | • | 2.218 | 2.059 | 1.764 |
| | •• | 100.0 | 92.8 | 79.5 |
| | ••• | 2.7 | 5.3 | 11.3 |
| | •••• | 2.9 | 2.9 | 2.9 |
| TF/23275 10 mg | • | 2.223 | 1.974 | 1.363 |
| | •• | 100.0 | 88.8 | 61.3 |
| | ••• | 2.8 | 11.3 | 15.1 |
| | •••• | 3.5 | 3.5 | 3.1 |
| 6001LA 10 mg | • | 2.155 | 1.984 | 1.667 |
| | •• | 100.0 | 92.1 | 77.4 |
| | ••• | 1.9 | 5.0 | 12.6 |
| | •••• | 3.0 | 3.0 | 3.0 |
| 6001LB 20 mg | • | 1.961 | 1.864 | 1.626 |
| | •• | 100.0 | 95.0 | 82.9 |
| | ••• | 1.2 | 5.9 | 10.7 |
| | •••• | 3.1 | 3.0 | 2.9 |
| 6001LC 50 mg | • | 2.072 | 2.118 | 2.002 |
| | •• | 100.0 | 102.2 | 96.6 |
| | ••• | 1.4 | 2.6 | 5.9 |
| | •••• | 2.7 | 2.7 | 2.6 |

•     epirubicin.HCl assay (mg/ml)
••    epirubicin.HCl % initial
•••   related substances %
••••  pH

Table 14- Stability data of epirubicin.HCl ready-to-use solution in 0.9% Sodium Chloride Injection, -20°C

Vials stored inverted

| Batch Dosage | | INITIAL CONTROL | 1 month | 3 months |
|---|---|---|---|---|
| TF/23274 10 mg | • | 2.218 | | |
| | •• | 100.0 | n.d. | n.d. |
| | ••• | 2.7 | | |
| | •••• | 2.9 | | |
| TF/23275 10 mg | • | 2.223 | | |
| | •• | 100.0 | n.d. | n.d. |
| | ••• | 2.8 | | |
| | •••• | 3.5 | | |
| 6001LA 10 mg | • | 2.155 | 2.185 | 2.131 |
| | •• | 100.0 | 101.4 | 98.9 |
| | ••• | 1.9 | 2.8 | 3.0 |
| | •••• | 3.0 | 3.0 | 3.0 |
| 6001LB 20 mg | • | 1.961 | 1.991 | 1.958 |
| | •• | 100.0 | 101.5 | 99.8 |
| | ••• | 1.2 | 3.0 | 3.3 |
| | •••• | 3.1 | 3.0 | 3.0 |
| 6001LC 50 mg | • | 2.072 | 2.049 | 2.184 |
| | •• | 100.0 | 98.9 | 105.4 |
| | ••• | 1.4 | 2.9 | 3.3 |
| | •••• | 2.7 | 2.7 | 2.7 |

•    epirubicin.HCl assay (mg/ml)
••   epirubicin.HCl % initial
•••  related substances %
•••• pH

n.d. = not determined

Table 15 - Stability data of epirubicin.HCl ready-to-use solution in 5% Dextrose solution, 4°C

<u>V i a l s   s t o r e d   u p r i g h t</u>

| Batch Dosage | | INITIAL CONTROL | 6 months |
|---|---|---|---|
| TF/23270 10 mg | • | 2.178 | |
| | •• | 100.0 | n.d. |
| | ••• | 3.7 | |
| | •••• | 2.7 | |
| TF/23273 10 mg | • | 2.132 | |
| | •• | 100.0 | n.d. |
| | ••• | 3.1 | |
| | •••• | 3.5 | |
| 600:MA 10 mg | • | 2.131 | 2.173 |
| | •• | 100.0 | 102.0 |
| | ••• | 2.0 | 4.0 |
| | •••• | 3.0 | 3.0 |
| 6001MB 20 mg | • | 1.973 | 1.917 |
| | •• | 100.0 | 97.5 |
| | ••• | 1.7 | 4.0 |
| | •••• | 3.1 | 3.1 |
| 6001MC 50 mg | • | 2.091 | 2.183 |
| | •• | 100.0 | 104.4 |
| | ••• | 1.1 | 3.3 |
| | •••• | 2.7 | 2.7 |

```
•     epirubicin.HCl assay (mg/ml)
••    epirubicin.HCl % initial
•••   related substances %
•••• pH
```

n.d. = not determined

Table 16 - Stability data of epirubicin.HCl ready-to-use solution in 5% Dextrose solution, 8°C

<u>V i a l s   s t o r e d   u p r i g h t</u>

| Batch Dosage | | INITIAL CONTROL | 1month | 3months | 6months |
|---|---|---|---|---|---|
| TF/23270 10 mg | • | 2.178 | | | |
| | •• | 100.0 | n.d. | n.d. | n.d. |
| | ••• | 3.7 | | | |
| | •••• | 2.9 | | | |
| TF/23273 10 mg | • | 2.132 | | | |
| | •• | 100.0 | n.d. | n.d. | n.d. |
| | ••• | 3.1 | | | |
| | •••• | 3.5 | | | |
| 6001MA 10 mg | • | 2.131 | 2.133 | 2.185 | 2.152 |
| | •• | 100.0 | 97.8 | 102.5 | 101.0 |
| | ••• | 2.0 | 2.9 | 3.1 | 4.4 |
| | •••• | 3.0 | 3.0 | 3.0 | 3.0 |
| 6001MB 20 mg | • | 1.973 | 1.979 | 1.936 | 1.934 |
| | •• | 100.0 | 100.3 | 98.1 | 98.4 |
| | ••• | 1.7 | 2.2 | 3.3 | 3.3 |
| | •••• | 3.1 | 3.1 | 3.0 | 3.1 |
| 6001MC 50 mg | • | 2.091 | 2.093 | 2.073 | 2.053 |
| | •• | 100.0 | 100.1 | 99.1 | 98.2 |
| | ••• | 1.1 | 3.3 | 2.5 | 3.3 |
| | •••• | 2.7 | 2.6 | 2.6 | 2.7 |

•    epirubicin.HCl assay (mg/ml)
••    epirubicin.HCl % initial
•••    related substances %
••••    pH

n.d. = not determined

Table 17 - Stability data of epirubicin.HCl ready-to-use
solution in 5% Dextrose solution, 4°C

<u>Vials stored inverted</u>

| Batch<br>Dosage | | INITIAL<br><u>CONTROL</u> | 6 months |
|---|---|---|---|
| TF/23270<br>10 mg | •<br>••<br>•••<br>•••• | 2.178<br>100.0<br>3.7<br>2.7 | 2.171<br>99.7<br>4.3<br>2.7 |
| TF/23273<br>10 mg | •<br>••<br>•••<br>•••• | 2.132<br>100.0<br>3.1<br>3.5 | 2.214<br>103.8<br>n.d.<br>3.5 |
| 6001MA<br>10 mg | •<br>••<br>•••<br>•••• | 2.131<br>100.0<br>2.0<br>3.0 | 2.241<br>105.2<br>3.6<br>3.0 |
| 6001MB<br>20 mg | •<br>••<br>•••<br>•••• | 1.973<br>100.0<br>1.7<br>3.1 | 1.899<br>96.6<br>2.9<br>3.1 |
| 6001MC<br>50 mg | •<br>••<br>•••<br>•••• | 2.091<br>100.0<br>1.1<br>2.7 | 2.128<br>101.8<br>3.0<br>2.7 |

•   epirubicin.HCl assay (mg/ml)
••   epirubicin.HCl % initial
•••   related substances %
•••• pH

Table 18 – Stability data of epirubicin.HCl ready-to-use
solution in 5% Dextrose solution, 8°C

<u>Vials stored inverted</u>

| Batch Dosage | | INITIAL CONTROL | 1 month | 3 months | 6 months |
|---|---|---|---|---|---|
| TF/23270 10 mg | • | 2.178 | 2.207 | 2.201 | 2.226 |
| | •• | 100.0 | 101.3 | 101.0 | 102.3 |
| | ••• | 3.7 | 3.9 | 3.8 | 2.6 |
| | •••• | 2.7 | 2.7 | 2.7 | 2.7 |
| TF/23273 10 mg | • | 2.132 | 2.189 | 2.143 | 2.149 |
| | •• | 100.0 | 102.7 | 100.5 | 100.8 |
| | ••• | 3.1 | 3.2 | 4.3 | 2.8 |
| | •••• | 3.5 | 3.5 | 3.4 | 3.5 |
| 6001MA 10 mg | • | 2.131 | 2.184 | 2.179 | 2.227 |
| | •• | 100.0 | 100.1 | 99.9 | 104.4 |
| | ••• | 2.0 | 3.2 | 3.0 | 3.6 |
| | •••• | 3.0 | 3.0 | 3.0 | 3.0 |
| 6001MB 20 mg | • | 1.973 | 1.964 | 1.913 | 1.893 |
| | •• | 100.0 | 99.5 | 97.0 | 96.3 |
| | ••• | 1.7 | 2.3 | 3.6 | 3.5 |
| | •••• | 3.1 | 3.0 | 3.0 | 3.1 |
| 6001MC 50 mg | • | 2.091 | 2.060 | 2.078 | 2.161 |
| | •• | 100.0 | 98.5 | 99.4 | 103.3 |
| | ••• | 1.1 | 2.9 | 2.9 | 3.5 |
| | •••• | 2.7 | 2.6 | 2.6 | 2.7 |

•     epirubicin.HCl assay (mg/ml)
••    epirubicin.HCl % initial
•••   related substances %
••••  pH

23

Table 19- Stability data of epirubicin.HCl  ready-to-use solution in 5% Dextrose solution,  15°C

Vials  stored  inverted

| Batch Dosage | | INITIAL CONTROL | 1 months | 3 months | 6 months |
|---|---|---|---|---|---|
| TF/23270 10 mg | • | 2.178 | 2.219 | 2.170 | 2.237 |
| | •• | 100.0 | 101.9 | 99.6 | 102.7 |
| | ••• | 3.7 | 3.4 | 4.3 | 2.7 |
| | •••• | 2.7 | 2.7 | 2.6 | 2.7 |
| TF/23273 10 mg | • | 2.132 | 2.153 | 2.079 | 2.119 |
| | •• | 100.0 | 101.0 | 97.5 | 99.4 |
| | ••• | 3.1 | 3.4 | 4.5 | n.d. |
| | •••• | 3.5 | 3.5 | 3.4 | 3.4 |
| 6001MA 10 mg | • | 2.131 | 2.158 | 2.155 | 2.133 |
| | •• | 100.0 | 98.9 | 98.8 | 100.1 |
| | ••• | 2.0 | 3.3 | 2.4 | 4.5 |
| | •••• | 3.0 | 3.0 | 3.0 | 3.0 |
| 6001MB 20 mg | • | 1.973 | 1.978 | 1.893 | |
| | •• | 100.0 | 100.2 | 95.9 | n.d. |
| | ••• | 1.7 | 2.0 | 3.7 | |
| | •••• | 3.1 | 3.1 | 3.0 | |
| 6001MC 50 mg | • | 2.091 | 2.121 | 2.066 | 2.061 |
| | •• | 100.0 | 101.4 | 98.8 | 98.5 |
| | ••• | 1.1 | 3.2 | 2.9 | 5.1 |
| | •••• | 2.7 | 2.6 | 2.7 | 2.7 |

•    epirubicin.HCl assay (mg/ml)

••   epirubicin.HCl % initial

•••  related substances  %

•••• pH

24

Table 20-Stability data of epirubicin.HCl ready-to-use solution in 5% Dextrose solution, 27°C

<u>V i a l s   s t o r e d   i n v e r t e d</u>

| Batch Dosage | | INITIAL CONTROL | 1 month | 3 months |
|---|---|---|---|---|
| TF/23270 10 mg | • | 2.178 | 2.113 | 2.052 |
| | •• | 100.0 | 97.0 | 94.2 |
| | ••• | 3.7 | 4.7 | 4.7 |
| | •••• | 2.7 | 2.7 | 2.7 |
| TF/23273 10 mg | • | 2.132 | 2.068 | 1.944 |
| | •• | 100.0 | 97.0 | 91.2 |
| | ••• | 3.1 | 6.0 | 7.5 |
| | •••• | 3.5 | 3.5 | 3.3 |
| 6001MA 10 mg | • | 2.131 | 2.144 | 2.033 |
| | •• | 100.0 | 96.9 | 93.2 |
| | ••• | 2.0 | 3.2 | 7.3 |
| | •••• | 3.0 | 3.0 | 3.0 |
| 6001MB 20 mg | • | 1.973 | 1.919 | 1.796 |
| | •• | 100.0 | 97.3 | 91.1 |
| | ••• | 1.7 | 2.1 | 6.2 |
| | •••• | 3.1 | 3.0 | 3.0 |
| 6001MC 50 mg | • | 2.091 | 2.096 | 1.896 |
| | •• | 100.0 | 100.6 | 90.7 |
| | ••• | 1.1 | 3.0 | 6.6 |
| | •••• | 2.7 | 2.6 | 2.6 |

• epirubicin.HCl assay (mg/ml)
•• epirubicin.HCl % initial
••• related substances %
•••• pH

Table 21 – Stability data of epirubicin.HCl ready-to-use solution in 5% Dextrose solution, 250 foot-candles

Vials stored inverted

| Batch Dosage | | INITIAL CONTROL | 1 months | 3 months |
|---|---|---|---|---|
| TF/23270 10 mg | • | 2.178 | 2.132 | 2.003 |
| | •• | 100.0 | 97.9 | 92.0 |
| | ••• | 3.7 | 6.7 | 9.2 |
| | •••• | 2.7 | 2.7 | 2.7 |
| TF/23273 10 mg | • | 2.132 | 2.046 | 1.796 |
| | •• | 100.0 | 96.0 | 84.2 |
| | ••• | 3.1 | 5.8 | 10.3 |
| | •••• | 3.5 | 3.4 | 3.2 |
| 6001MA 10 mg | • | 2.131 | 2.079 | 1.988 |
| | •• | 100.0 | 95.3 | 91.1 |
| | ••• | 2.0 | 3.7 | 8.2 |
| | •••• | 3.0 | 3.0 | 3.0 |
| 6001MB 20 mg | • | 1.973 | 1.894 | 1.803 |
| | •• | 100.0 | 96.0 | 91.4 |
| | ••• | 1.7 | 2.0 | 5.6 |
| | •••• | 3.1 | 3.0 | 3.0 |
| 6001MC 50 mg | • | 2.091 | 2.022 | 1.988 |
| | •• | 100.0 | 96.7 | 95.1 |
| | ••• | 1.1 | 4.0 | 4.2 |
| | •••• | 2.7 | 2.6 | 2.6 |

• epirubicin.HCl assay (mg/ml)
•• epirubicin.HCl % initial
••• related substances %
•••• pH

Table 22 Stability data of epirubicin.HCl ready-to-use solution in 5% Dextrose solution, -20°C

Vials stored inverted

| Batch Dosage | | INITIAL CONTROL | 1month | 3 months |
|---|---|---|---|---|
| TF/23270 10 mg | • | 2.178 | | |
| | •• | 100.0 | n.d. | n.d. |
| | ••• | 3.7 | | |
| | •••• | 2.7 | | |
| TF/23273 10 mg | • | 2.132 | | |
| | •• | 100.0 | n.d. | n.d. |
| | ••• | 3.1 | | |
| | •••• | 3.5 | | |
| 6001MA 10 mg | • | 2.131 | 2.168 | 2.162 |
| | •• | 100.0 | 99.4 | 99.1 |
| | ••• | 2.0 | 2.2 | 3.3 |
| | •••• | 3.0 | 3.0 | 3.0 |
| 6001MB 20 mg | • | 1.973 | 1.985 | 1.994 |
| | •• | 100.0 | 100.6 | 101.4 |
| | ••• | 1.7 | 2.6 | 3.8 |
| | •••• | 3.1 | 3.0 | 3.0 |
| 6001MC 50 mg | • | 2.091 | 2.114 | 2.090 |
| | •• | 100.0 | 101.9 | 99.9 |
| | ••• | 1.1 | 2.5 | 2.6 |
| | •••• | 2.7 | 2.6 | 2.6 |

•   epirubicin.HCl assay (mg/ml)
••  epirubicin.HCl % initial
••• related substances %
•••• pH

n.d. = not determined

Table 23 - Stability data of epirubicin.HCl ready-to-use solution in Water for Injection Batch TF/23176

<u>V i a l s   s t o r e d   u p r i g h t</u>

| Storage temperature | Tests | 0 | 1 | 2 | 3 | Time (months) 6 | 9 - | 12 |
|---|---|---|---|---|---|---|---|---|
| – 20°C | • | 2.079 | 2.061 | | 2.059 | | | |
| | •• | 100.0 | 99.1 | n.d. | 99.0 | n.d. | n.d. | |
| | ••• | 4.0 | 3.8 | | 4.9 | | | |
| | •••• | 3.0 | 3.1 | | 3.2 | | | |
| 4°C | • | 2.079 | | | 2.062 | 2.061 | | 1.976 |
| | •• | 100.0 | n.d. | n.d. | 99.2 | 99.1 | n.d. | 95.0 |
| | ••• | 4.0 | | | 3.7 | 3.4 | | 6.7 |
| | •••• | 3.0 | | | 3.2 | 3.2 | | 3.1 |
| 8°C | • | 2.079 | 2.041 | 2.043 | 1.995 | 1.986 | | 1.950 |
| | •• | 100.0 | 98.2 | 98.3 | 96.0 | 95.5 | n.d. | 93.8 |
| | ••• | 4.0 | 3.6 | 3.8 | 4.0 | 3.4 | | 6.7 |
| | •••• | 3.0 | 3.0 | 3.0 | 3.1 | 3.2 | | 3.1 |
| 15°C | • | 2.079 | 2.001 | 1.989 | 1.990 | 1.923 | | 1.757 |
| | •• | 100.0 | 96.2 | 95.7 | 95.7 | 92.5 | n.d. | 84.5 |
| | ••• | 4.0 | 3.7 | 3.9 | 3.9 | 3.5 | | 8.8 |
| | •••• | 3.0 | 3.0 | 3.0 | 3.1 | 3.2 | | 3.1 |
| 27°C | • | 2.079 | 1.977 | 1.879 | 1.830 | 1.480 | | |
| | •• | 100.0 | 95.1 | 90.4 | 88.0 | 71.2 | n.d. | |
| | ••• | 4.0 | 5.0 | 6.8 | 6.9 | 9.0 | | |
| | •••• | 3.0 | 3.0 | 3.1 | 3.1 | 3.1 | | |

• epirubicin.HCl assay (mg/ml)
•• epirubicin.HCl % initial
••• related substances %
•••• pH

n.d. = not determined

Table 24 - Stability data of epirubicin.HCl ready-to-use
solution in Water for Injection Batch TF/23176

V i a l s    s t o r e d    i n v e r t e d

| Storage temperature | Tests | 0 | 1 | 2 | 3 | Time (months) 6 | 9 | 12 |
|---|---|---|---|---|---|---|---|---|
| – 20°C | • | 2.079 | 2.052 | | 2.060 | | | |
| | •• | 100.0 | 98.7 | n.d. | 99.1 | n.d. | n.d. | n.d. |
| | ••• | 4.0 | 3.7 | | 4.6 | | | |
| | •••• | 3.0 | 3.1 | | 3.2 | | | |
| 4°C | • | 2.079 | | | 2.045 | 2.067 | | |
| | •• | 100.0 | n.d. | n.d. | 98.4 | 99.4 | n.d. | n.d. |
| | ••• | 4.0 | | | 3.6 | 2.9 | | |
| | •••• | 3.0 | | | 3.2 | 3.2 | | |
| 8°C | • | 2.079 | 2.033 | 2.039 | 2.022 | 1.977 | | |
| | •• | 100.0 | 97.8 | 98.1 | 97.3 | 95.1 | n.d. | n.d. |
| | ••• | 4.0 | 3.5 | 3.8 | 3.7 | 3.1 | | |
| | •••• | 3.0 | 3.0 | 3.0 | 3.1 | 3.2 | | |
| 15°C | • | 2.079 | 1.992 | 1.994 | 1.994 | 1.934 | | 1.759 |
| | •• | 100.0 | 95.8 | 95.9 | 95.9 | 93.0 | n.d. | 84.6 |
| | ••• | 4.0 | 3.6 | 3.9 | 4.6 | 4.4 | | 8.6 |
| | •••• | 3.0 | 3.0 | 3.0 | 3.1 | 3.2 | | 3.1 |
| 27°C | • | 2.079 | 1.964 | 1.836 | 1.779 | 1.566 | | |
| | •• | 100.0 | 94.5 | 88.3 | 85.6 | 75.4 | | |
| | ••• | 4.0 | 5.0 | 7.9 | 6.9 | 7.4 | | |
| | •••• | 3.0 | 3.0 | 3.1 | 3.1 | 3.1 | | |
| R.T.+ 100 F.C. | • | 2.079 | 1.923 | | 1.778 | | | |
| | •• | 100.0 | 92.5 | n.d. | 85.5 | | | |
| | ••• | 4.0 | 4.8 | | 7.5 | | | |
| | •••• | 3.0 | 3.1 | | 3.1 | | | |
| R.T.+ 250 F.C. | • | 2.079 | 1.886 | | 1.473 | | | |
| | •• | 100.0 | 90.7 | n.d. | 71.8 | | | |
| | ••• | 4.0 | 6.6 | | 13.5 | | | |
| | •••• | 3.0 | 3.0 | | 3.0 | | | |

•       epirubicin.HCL assay (mg/ml)
••      epirubicin.HCl % initial
•••     related substances %
••••    pH

n.d. = not determined
R.T. = room temperature
F.C. = foot candles

**Claims**
**Claims for the following Contracting States : AT, BE, CH, DE, FR, GB, IT, LU, NL, SE**

1. A storage stable, sterile, pyrogen-free, ready-to-use, injectable 4'-epi-doxorubicin solution which is sealed in a container, which consists essentially of 4'-epi-doxorubicin hydrochloride dissolved in a physiologically acceptable aqueous solvent therefor at a concentration of 4'-epi-doxorubicin of from 0.1 mg/ml to 50 mg/ml, which has not been reconstituted from a lyophilizate and the pH of which has been adjusted to from 2.5 to 4.0 solely by means of a physiologically acceptable acid.

2. A solution according to claim 1 wherein the said acid is an inorganic mineral acid.

3. A solution according to claim 2 wherein the acid is hydrochloric, sulfuric or phosphoric acid.

4. A solution according to claim 1 wherein the said acid is an organic acid.

5. A solution according to claim 4, wherein the acid is acetic, succinic, tartaric, ascorbic, citric, glutamic, methanesulphonic or ethanesulphonic acid.

6. A solution according to any one of the preceding claims containing dextrose, lactose, sorbitol or mannitol as a tonicity adjustment agent.

7. A solution according to any one of the preceding claims, wherein the physiologically acceptable solvent is water or physiological saline or an aqueous 5% dextrose solution.

8. A solution according to any one of the preceding claims having a pH of about 3.

9. A process for producing the sealed, storage-stable, sterile, pyrogen-free, injectable solution of any one of the preceding claims, which process comprises
   (i) dissolving the 4'-epi-doxorubicin hydrochloride, which is not in the form of a lyophilizate, in the physiologically acceptable aqueous solvent therefor at a concentration of 4'-epi-doxorubicin of from 0.1 mg/ml to 50 mg/ml;
   (ii) optionally, adding one or more formulation adjuvants selected from co-solubilizing agents, tonicity adjustment agents, preservatives and pharmaceutically acceptable chelating agents;
   (iii) adding solely a physiologically acceptable acid to adjust the pH to from 2.5 to 4.0 as desired;
   (iv) sealing the solution in a container; and the process being effected in such a manner that the resultant solution is sterile and pyrogen-free.

10. A process according to claim 9 wherein the solution is passed through a sterilising filter after step (iii) but before step (iv).

**Claims for the following Contracting States : ES, GR**

1. A process for producing a sealed, storage-stable, sterile, pyrogen-free, ready-to-use injectable solution of 4'-epi-doxorubicin, which process comprises:
   (i) dissolving 4'-epi-doxorubicin hydrochloride, which is not in the form of a lyophilizate, in a physiologically acceptable aqueous solvent therefor at a concentration of 4'-epi-doxorubicin of from 0.1 mg/ml to 50 mg/ml;
   (ii) optionally, adding one or more formulation adjuvants selected from co-solubilizing agents, tonicity adjustment agents, preservatives and pharmaceutically acceptable chelating agents;
   (iii) adding solely a physiologically acceptable acid to adjust the pH to from 2.5 to 4.0 as desired;
   (iv) sealing the solution in a container; and the process being effected in such a manner that the resultant solution is sterile and pyrogen-free.

2. A process according to claim 1, wherein the solution is passed through a sterilizing filter after step (iii) but before step (iv).

3. A process according to claim 1 or 2, wherein the said acid is an inorganic mineral acid.

4. A process according to claim 3, wherein the acid is hydrochloric, sulfuric or phosphoric acid.

5. A process according to claim 1 or 2, wherein the said acid is an organic acid.

6. A process according to claim 5, wherein the acid is acetic, succinic, tartaric, ascorbic, citric, glutamic, methanesulphonic or ethanesulphonic acid.

7. A process according to any one of the preceding claims containing dextrose, lactose, sorbitol or mannitol as a tonicity adjustment agent.

8. A process according to any one of the preceding claims, wherein the physiologically acceptable solvent is water or physiological saline or an aqueous 5 % dextrose solution.

9. A process according to any one of the preceding claims, wherein the pH is adjusted to about 3.

**Patentansprüche**
**Patentansprüche für folgende Vertragsstaaten : AT, BE, CH, DE, FR, GB, IT, LU, NL, SE**

1. Lagerstabile, sterile, pyrogenfreie, gebrauchsfertige, injizierbare Lösung von 4'-Epi-doxorubicin, die in einem Behälter verschlossen ist, bestend im wesentlichen aus 4'-Epi-doxorubicinhydrochlorid, gelöst in einem physiologisch annehmbaren wäßrigen Lösungsmittel dafür in einer Konzentration von 4'-Epi-doxorubicin von 0,1 mg/ml bis 50 mg/ml, die nicht aus einem Lyophilisat rekonstituiert ist, und deren pH-Wert auf 2,5 bis 4,0 allein mittels einer physiologisch annehmbaren Säure eingestellt worden ist.

2. Lösung gemäß Anspruch 1, in welcher die Säure eine anorganische Mineralsäure ist.

3. Lösung gemäß Anspruch 2, in welcher die Säure Salzsäure, Schwefelsäure oder Phosphorsäure ist.

4. Lösung gemäß Anspruch 1, in welcher die Säure eine organische Säure ist.

5. Lösung gemäß Anspruch 4, in welcher die Säure Essigsäure, Bernsteinsäure, Weinsäure, Ascorbinsäure, Zitronensäure, Glutanimsäure, Methansulfonsäure oder Ethansulfonsäure ist.

6. Lösung gemäß einem der vorhergehenden Ansprüche, enthaltend Dextrose, Lactose, Sorbit oder Mannit als Tonizitätseinstellungsmittel.

7. Lösung gemäß einem der vorhergehenden Ansprüche, in welcher das physiologisch annehmbare Lösungsmittel Wasser oder eine physiologische Salzlösung oder eine wäßrige 5 %igen Dextroselösung ist.

8. Lösung gemäß einem der vorhergehenden Ansprüche mit einem pH-Wert von etwa 3.

9. Verfahren zur Herstellung der verschlossenen, lagerstabilen, sterilen, pyrogenfreien, injizierbaren Lösung gemäß einem der vorhegehenden Ansprüche, umfassend
    (i) Auflösen von 4'-Epi-doxorubicinhydrochlorid, das nicht in Form eines Lyophilisats vorliegt, in einem physiologisch annehmbaren, wäßrigen Lösungsmittel dafür, in einer Konzentration des 4-Epidoxorubicins von 0,1 mg/ml bis 50 mg/ml;
    (ii) gewünschtenfalls Zugeben einer oder mehrerer Formulierungen von Adjuvantien, ausgewählt aus Co-Solubilisierungsmittel, Tonizitäts-Einstellungsmitteln, Konservierungsmitteln und pharmazeutisch annehmbaren Gelatbildnern;
    (iii) alleiniges Zugeben einer physiologisch annehmbaren Säure zur Einstellung des gewünschten pH-Wertes auf 2,5 bis 4,0;
    (iv) Verschließen der Lösung in einen Behälter, wobei das Verfahren so durchgeführt wird, daß die erhaltene Lösung steril und pyrogenfrei ist.

10. Verfahren gemäß Anspruch 9, bei dem man die Lösung durch einen Sterilisierungsfilter nach der Stufe (iii) aber vor der Stufe (iv) durchlaufen läßt.

**Patentansprüche für folgende Vertragsstaaten : ES, GR**

1. Verfahren zur Herstellung einer verschlossenen, lagerstabilen, sterilen, pyrogenfreien, gebrauchsfertigen, injizierbaren Lösung von 4'-Epi-doxorubicin, umfassend:

   (i) Auflösen von 4'-Epi-doxorubicinhydrochlorid, das nicht in Form eines Lyophilisats vorliegt, in einem physiologisch annehmbaren, wäßrigen Lösungsmittel dafür, in einer Konzentration des 4-Epidoxorubicins von 0,1 mg/ml bis 50 mg/ml;

   (ii) gewünschtenfalls Zugeben einer oder mehrerer Formulierungen von Adjuvantien, ausgewählt aus Co-Solubilisierungsmittel, Tonizitäts-Einstellungsmitteln, Konservierungsmitteln und pharmazeutisch annehmbaren Gelatbildnern;

   (iii) alleiniges Zugeben einer physiologisch annehmbaren Säure zur Einstellung des gewünschten pH-Wertes auf 2,5 bis 4,0;

   (iv) Verschließen der Lösung in einen Behälter, wobei das Verfahren so durchgeführt wird, daß die erhaltene Lösung steril und pyrogenfrei ist.

2. Verfahren gemäß Anspruch 1, bei den man die Lösung durch einen Sterilisierungsfilter nach der Stufe (iii) aber vor der Stufe (iv) durchlaufen läßt.

3. Verfahren gemäß Ansprüchen 1 oder 2, bei dem die Säure eine anorganische Mineralsäure ist.

4. Verfahren gemäß Anspruch 3, bei dem die Säure Salzsäure, Schwefelsäure oder Phosphorsäure ist.

5. Verfahren gemäß Ansprüchen 1 oder 2, bei dem die Säure eine organische Säure ist.

6. Verfahren gemäß Anspruch 5, bei dem die organische Säure Essigsäure, Bernsteinsäure, Weinsäure, Ascorbinsäure, Zitronensäure, Glutaminsäure, Methansulfonsäure oder Ethansulfonsäure ist.

7. Verfahren gemäß einem der vorhergehenden Ansprüche, bei dem Dextrose, Lactose, Sorbit oder Mannit als Tonizität-Einstellungsmittel enthalten sind.

8. Verfahren gemaß einem der vorhergehenden Ansprüche, bei dem das physiologisch annehmbare Lösungsmittel Wasser oder eine physiologische Salzlösung oder eine wäßrige 5 %ige Dextroselösung ist.

9. Verfahren gemäß einem der vorhergehenden Ansprüche, bei dem der pH-Wert auf etwa 3 eingestellt wird.

**Revendications**

**Revendications pour les Etats contractants suivants : AT, BE, CH, DE, FR, GB, IT, LU, NL, SE**

1. Solution de 4'-épi-doxorubicine, injectable, prête à l'emploi, exempte de pyrogènes, stérile et stable au stockage, qui est enfermée de manière étanche dans un récipient, est essentiellement constituée de chlorhydrate de 4'-épi-doxorubicine dissous dans un solvant aqueux physiologiquement acceptable de celui-ci, à une concentration de 0,1 mg/ml à 50 mg/ml de 4'-épi-doxorubicine, qui n'a pas été reconstituée à partir d'un lyophilisat, et dont le pH a été ajusté à 2,5 - 4,0 uniquement au moyen d'un acide physiologiquement acceptable.

2. Solution selon la revendication 1, dans laquelle ledit acide est un acide minéral inorganique.

3. Solution selon la revendication 2, dans laquelle l'acide est l'acide chlorhydrique, l'acide sulfurique ou l'acide phosphorique.

4. Solution selon la revendication 1, dans laquelle ledit acide est un acide organique.

5. Solution selon la revendication 4, dans laquelle l'acide est l'acide acétique, l'acide succinique, l'acide tartrique, l'acide ascorbique, l'acide citrique, l'acide glutamique, l'acide méthanesulfonique ou l'acide éthanesulfonique.

6. Solution selon l'une quelconque des revendications précédentes, contenant du dextrose, du lactose, du sorbitol ou du mannitol comme agents d'ajustement de la tonicité.

7. Solution selon l'une quelconque des revendications précédentes, dans laquelle le solvant physiologiquement acceptable est de l'eau, une solution saline physiologique ou une solution aqueuse à 5% de dextrose.

8. Solution selon l'une quelconque des revendications précédentes, présentant un pH d'environ 3.

9. Procédé de production de la solution injectable, exempte de pyrogènes, stérile, stable au stockage et enfermée de manière étanche, selon l'une quelconque des revendications précédentes, ce procédé comportant les étapes consistant à:
   (i) dissoudre le chlorhydrate et de 4'-épi-doxorubicine, qui ne se présente pas sous la forme d'un lyophilisat, dans le solvant aqueux physiologiquement acceptable de celui-ci, à une concentration de 0,1 mg/ml à 50 mg/ml de 4'-épi-doxorubicine;
   (ii) facultativement, ajouter un ou plusieurs adjuvants de formulation choisis parmi des agents de co-solubilisation, des agents d'ajustement de la tonicité, des agents de conservation et des agents chélatants pharmaceutiquement acceptables;
   (iii) ajouter uniquement un acide physiologiquement acceptable pour ajuster le pH à 2,5 - 4,0, comme souhaité;
   (iv) enfermer la solution de manière étanche dans un récipient;
   procédé étant réalisé de telle sorte que la solution résultante soit stérile et exempte de pyrogènes.

10. Procédé selon la revendication 9, dans lequel la solution est passée à travers un filtre de stérilisation après l'étape (iii) mais avant l'étape (iv).

**Revendications pour les Etats contractants suivants : ES, GR**

1. Procédé de production d'une solution de 4'-épi-doxorubicine, injectable, prête à l'emploi exempte de pyrogènes, stérile, stable au stockage et enfermée de manière étanche, ce procédé comportant les étapes consistant à:
   (i) dissoudre du chlorhydrate et de 4'-épi-doxorubicine, qui ne se présente pas sous la forme d'un lyophilisat, dans un solvant aqueux physiologiquement acceptable de celui-ci, à une concentration de 0,1 mg/ml à 50 mg/ml de 4'-épi-doxorubicine;
   (ii) facultativement, ajouter un ou plusieurs adjuvants de formulation choisis parmi des agents de co-solubilisation, des agents d'ajustement de la tonicité, des agents de conservation et des agents chélatants pharmaceutiquement acceptables;
   (iii) ajouter uniquement un acide physiologiquement acceptable pour ajuster le pH à 2,5 - 4,0, comme souhaité;
   (iv) enfermer la solution de manière étanche dans un récipient;
   le procédé étant réalisé de telle sorte que la solution résultante soit stérile et exempte de pyrogènes.

2. Procédé selon la revendication 1, dans lequel la solution est passée à travers un filtre de stérilisation après l'étape (iii) mais avant l'étape (iv).

3. Procédé selon la revendication 1 ou 2, dans lequel ledit acide est un acide minéral inorganique.

4. Procédé selon la revendication 3, dans lequel l'acide est l'acide chlorhydrique, l'acide sulfurique ou l'acide phosphorique.

5. Procédé selon la revendication 1 ou 2, dans lequel ledit acide est un acide organique.

6. Procédé selon la revendication 5, dans lequel l'acide est l'acide acétique, l'acide succinique, l'acide tartrique, l'acide ascorbique, l'acide citrique, l'acide glutamique, l'acide méthanesulfonique ou l'acide éthanesulfonique.

7. Procédé selon l'une quelconque des revendications précédentes, contenant du dextrose, du lactose, du sorbitol ou du mannitol comme agent d'ajustement de la tonicité.

8. Procédé selon l'une quelconque des revendications précédentes, dans lequel le solvant physiologiquement acceptable est de l'eau, une solution saline physiologique ou une solution aqueuse à 5% de dextrose .

9. Procédé selon l'une quelconque des revendications précédentes, dans lequel le pH est ajusté à environ 3.